(19) Europäisches Patentamt
European Patent Office
Office européen des brevets

(11) Publication number : 0 518 798 A2

(12) # EUROPEAN PATENT APPLICATION

(21) Application number : 92470014.9

(22) Date of filing : 09.04.92

(51) Int. Cl.$^5$ : A61K 31/33

(30) Priority : 09.04.91 US 682347

(43) Date of publication of application :
16.12.92 Bulletin 92/51

(84) Designated Contracting States :
AT BE CH DE DK ES FR GB GR IT LI LU MC NL PT SE

(71) Applicant : CHEMEX/BLOCK DRUG, JV
257 Cornelison Avenue
Jersey City, NJ 07302 (US)

(72) Inventor : Vora, Kabubhai Rashmi
924, Pennsylvania Avenue
Westfield, NJ 07090 (US)
Inventor : Khandwala, Atul
1055 River Road, Unit 310
Edgewater, NJ 07024 (US)
Inventor : Smith, Charles G.
17323 Circa-Del Sur
Rancho, Sante Fe, CA 92067 (US)

(74) Representative : Poupon, Michel
B.P. 421 3, rue Ferdinand Brunot
F-88011 Epinal Cédex (FR)

(54) **Compositions for use in the treatment of aphtous ulcers and other mucocutaneous disorders.**

(57) A method of treating aphthous ulcers and other mucocutaneous disorders is disclosed. The method comprises contacting the mucocutaneous disorder with a composition in the form of a paste, solution, gel, quick-disintegrating table, mouthwash, ointment, cream, powder, adhesive patch, aerosolized spray, lozenge, troche, dentifrice, or dental floss that contains an effective amount of one or more compounds having the following biochemical/biological properties : mediator release inhibitors, 5-lipoxygenase inhibitors, leukotriene antgonists, and platelet-activating factor antagonists.

EP 0 518 798 A2

## BACKGROUND OF THE INVENTION

### 1. Field of the Invention

The present invention relates to a method for treating aphthous ulcers and other mucocutaneous disorders.

### 2. Background of the Invention

Aphthous ulcers, often referred to as canker sores, are characterized by painful eruptions in the mucous membrane of the mouth. Of unknown etiology, they are covered by a grey exudate, and surrounded by a reddened area. They range in size from several millimeters to two centimeters in diameter. The ulcers are limited to oral mucous membranes not bound to periosteum, e.g. the inner portion of the lip or cheek. Aphthous ulcers may occur as solitary or multiple lesions, and heal spontaneously in one or two weeks. (Steadman's Medical Dictionary, 25th Ed., Williams & Wilkins)

Other mucocutaneous disorders can also result in the formation of oral ulcers that can be extremely painful.

Therapy for mouth ulcers generally involves use of topical anesthetics such as benzocaine in preparations made with a carrier designed to protect the ulcer from saliva and hold the anesthetic at the site. Zilactin is a topical medication composed of hydroxypropylcellulose, salicylic, lauric and tannic acids which has mucousal adherence properties. The mode of action of the product appears to be its effective film-forming capability that insulates the ulcer from the mouth environment. Because this characteristic requires effective formation of such a film, the product is difficult to apply in a manner sufficient to optimize its effect.

Still lacking is a method of treating apthous ulcers and other mucocutaneous disorders which is easier to apply and which actually speeds the healing of the ulcers. These objectives are achieved by the present invention, which involves the use of 5-lipoxygenase inhibitors, leukotriene antagonists and platelet-activating factor ("PAF") antagonists.

Compounds with one or more of the biochemical/biological activities identified above are to known to have antiallergic and antiinflammatory activities. (Musser et al., Ann. Reports in Medicinal Chemistry, 93-105 (1984); Saunders & Handley, Ann. Rev. Pharmacol. Toxicol., 27, 237-55 (1987). These compounds are of value as prophylactic and curative drugs for the treatment of allergic asthma, allergic dermatitis, hay fever and other allergic diseases in mammals, including humans. However, none of these substances have been reported as possessing anti-ulcer activity. In Chemical Marketing Reporter, August 14, 1989 is was reported that tests were underway to test use of amlexanox, which may have one or more of the aforementioned biochemical/biological properties, for treatment of mouth ulcers. No results were provided.

## SUMMARY OF THE INVENTION

It is the principal object of the present invention to provide a novel and effective method of treating aphthous ulcers and other mucocutaneous disorders.

Another object of the present invention is to provide a method of treating aphthous ulcers and other mucocutaneous disorders in various dosage forms that are convenient to use.

Another object of the invention is to provide a method of treating aphthous ulcers and other mucocutaneous disorders in dosage forms that can be applied at a specific site in the oral cavity with a finger tip or which can be easily masticated for contact with the oral mucosa.

An additional objective of the invention is to provide a method of treating aphthous ulcers and other mucocutaneous disorders in formulations with various release rates for greater efficacy.

To achieve the foregoing objectives and in accordance with the purposes of the invention as embodied and broadly described herein, there is provided a method of treating aphthous ulcers and other mucocutaneous disorders comprising contacting the aphthous ulcer or other mucocutaneous disorder with a composition containing an effective amount of a compound having one or more or the following biochemical/biological properties: mediator release inhibitors, 5-lipoxygenase inhibitors, leukotriene antagonists and PAF antagonists.

## DETAILED DESCRIPTION OF THE INVENTION

In this application "treating agent" is one or more compounds that can be used for the treatment of aphthous ulcers or other mucocutaneous disorders having one or more of the following biochemical/biological properties: mediator release inhibitors, 5-lipoxygenase inhibitors, leukotriene antagonists, and PAF antagonists.

Mediator release inhibitors include but are not limited to the following compounds:

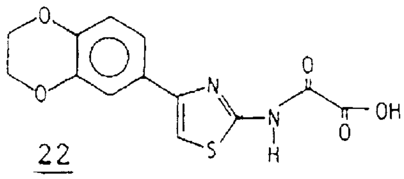
22

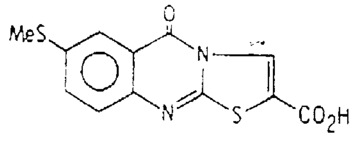
23

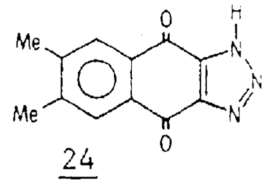
24

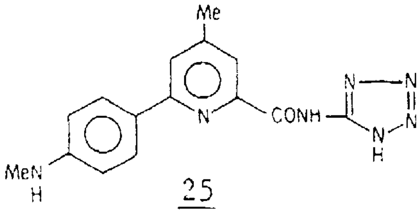
25

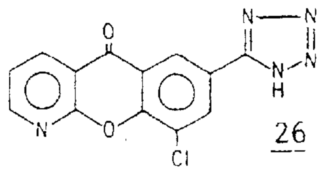
26

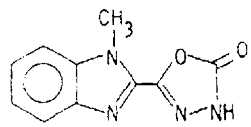
27

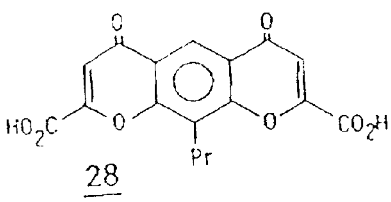
28

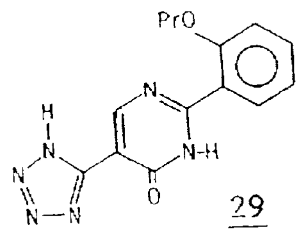
29

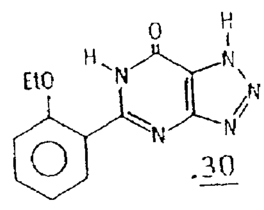
30

5-lipoxygenase inhibitors include but are not limited to the following compounds:

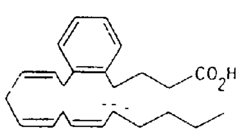
1

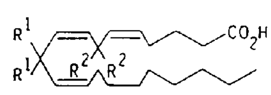
2 a) $R^2 = CH_3$, $R^1 = H$
b) $R^1 = CH_3$, $R^2 = H$

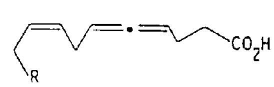
3 R = 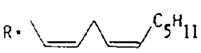
4 R = $C_{10}H_{21}$

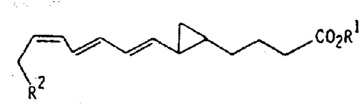
5 $R^2$ = 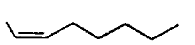 , $R^1 = H$
6 $R^2$ = 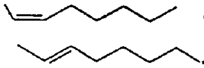 , $R^1 = CH_3$

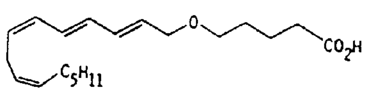
7

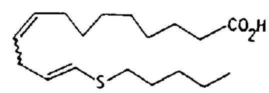
8

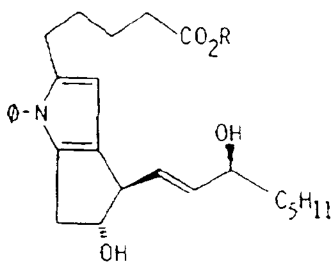

9

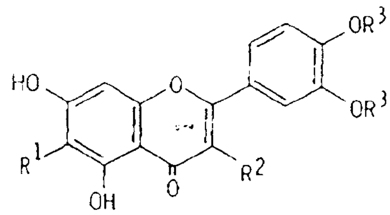

10 $R^1 = R^3 = H$, $R^2 = OH$

11 $R^1 = OCH_3$, $R^2 = H$, $R^3 = CH_3$

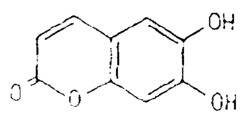

12

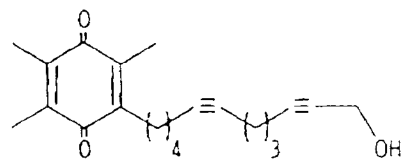

13

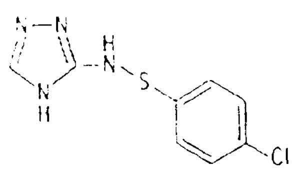

14

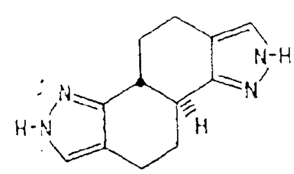

15

Leukotriene antagonists include but are not limited to the following compounds:

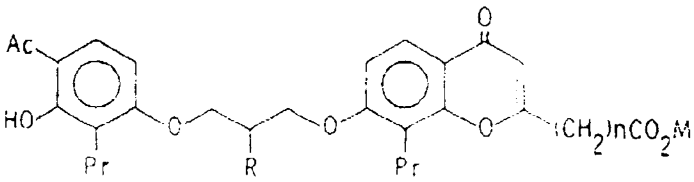

16 R=OH n=0 M=Na
17 R=H n=2 M=(Lysine)H

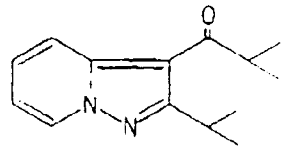

18

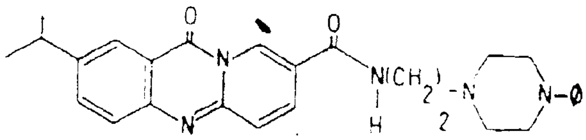

19

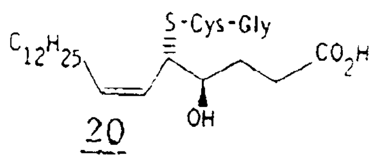

20

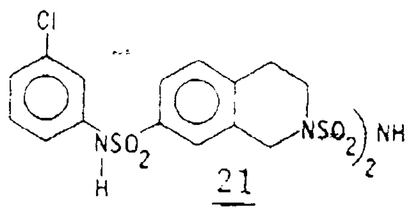

21

PAF antagonists include but are not limited to the following compounds:

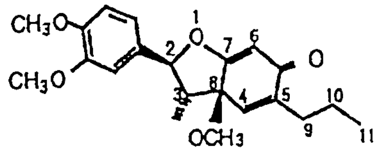

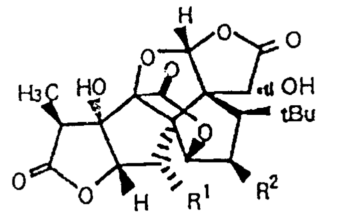

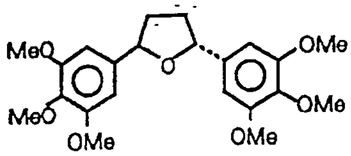

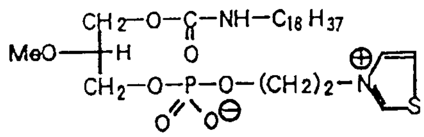

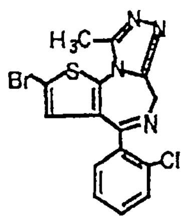

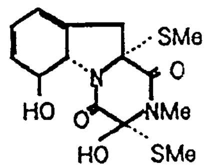

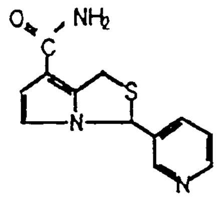

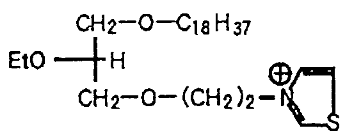

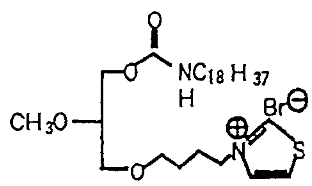

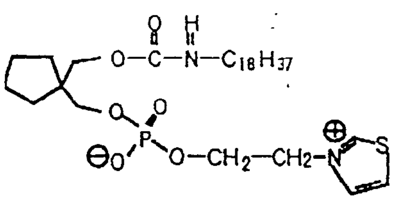

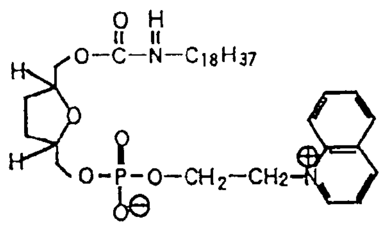

Chemical nomenclature relating to SRI 63-119, SRI 63-441, SRI 63-072 ONO 6240 and CV 3988 will be found at page 6a.

Improved drawings relating to Brotizolam, Kadsurenone and BN-52021 will be found at page 6b.

1
CHEMLINE
Registry Number: 102841-48-5
C30-H57-N2-O4-S.Br
Thiazolium, 3-(4-(2-methoxy-3-(((octadecylamino)carbonyl)oxy)propoxy)butyl)-, bromide, (+-)- (9CI) [MF1]

Synonyms:

SRI 63-119 [CAS]

6
CHEMLINE
Registry Number: 104786-62-1
C36-H59-N2-O7-P
DL-erythro-Hexitol, 2,5-anhydro-3,4-dideoxy-, octadecylcarbamate 2-quinolinioethyl hydrogen phosphate, hydroxide, inner salt (9CI)

Synonyms:

SRI 63441 [CAS]

7
CHEMLINE
Registry Number: 102841-49-6
C30-H55-N2-O8-P-S
Thiazolium, 4-hydroxy-3-(2-((hydroxy((tetrahydro-2-((((octadecylamino)carbony-l)oxy)methyl)-2-furanyl)methoxy)phosphinyl)oxy)ethyl)-, hydroxide, inner salt, (+-)- (9CI)

Synonyms:

SRI 63-072 [CAS]

1
CHEMLINE
Registry Number: 99659-62-8
C31-H60-N-O3-S.C-H3-O3-S
Thiazolium, 3-(7-(2-ethoxy-3-(hexadecyloxy)propoxy)heptyl)-, (+-)-, methanesulfonate (9CI) [MF1]

Synonyms:

ONO 6240 [CAS]

13
CHEMLINE
Registry Number: 85703-73-7
C28-H53-N2-O7-P-S
Thiazolium, 3-(4-hydroxy-7-methoxy-10-oxo-3,5,9-trioxa-11-aza-4-phosphanonaco-s-1-yl)-, hydroxide, inner salt, P-oxide, (+-)- (9CI)

Synonyms:

CV 3988 [CAS]

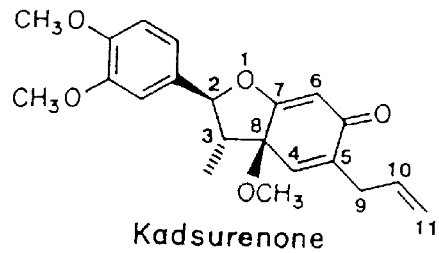

Kadsurenone

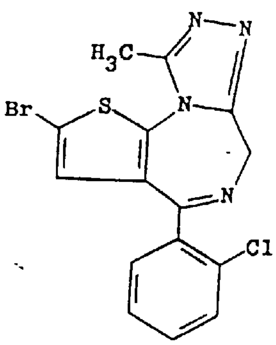

**Brotizolam**

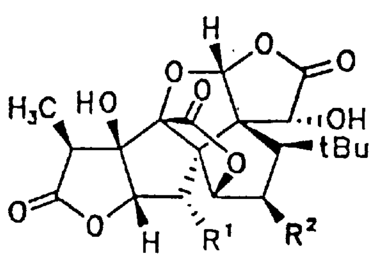

| | | |
|---|---|---|
| BN 52020 | $R^1$ - H | $R^2$ - H |
| BN 52021 | $R^1$ - OH | $R^2$ - H |
| BN 52022 | $R^1$ - OH | $R^2$ - OH |

Another group of compounds that can be used as a "treating agent" is amlexanox and its homologues and analogues. As disclosed in U.S. Pat. No. 4,143,042, amlexanox is a compound of the formula 2-amino-7-(1-methylethyl)-5-oxo-5H-[1]benzopyrano (2,3-b)pyridine-3-carboxylic acid. Amlexanox and its homologues and analogues are known to have anti-allergic activity, and are of value as prophylactic and curative drugs for the treatment of allergic asthma, allergic dermatitis, hay fever and other allergic diseases in mammals, including humans. However, they have never been reported to possess anti-ulcer activity.

Amlexanox and its homologues and analogues are compounds of the formula:

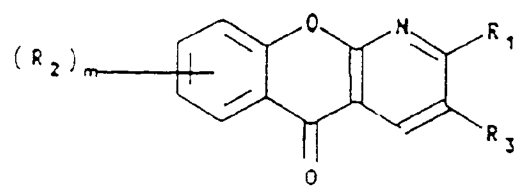

wherein $R_1$ is hydrogen, alkyl, phenyl, carboxyl, hydroxyl, alkoxyl, carboxyalkyl (i.e. esters), cyano, acylamino, or amino group which may be unsubstituted or substitued by up to two alkyl groups; m is 0, 1 or 2 and $R_2$ is alkyl, alkenyl, alkoxy, halogen, nitro, hydroxy, carboxyl, butadienylene (-CH=CH-CH=CH-) which forms a benzene ring with any adjacent carbon atoms, cyano, carboxyalkyl, trifluoromethyl, or amino group which may be unsubstituted or substitued by at least one alkyl; and $R_3$ is carboxyl, cyano, arylalkoxycarbonyl, alkoxycarbonyl, or carboxamide which may be unsubstituted or substitued by at least one alkyl, and the salts thereof.

Dosage forms suitable for delivering one or mode drugs to the oral mucosal membrane may include paste, solution, gel, quick-disintegrating tablet, mouthwash, ointment, cream, powder, adhesive patch, aerosolized spray, lozenge, troche, dentifrice and dental floss. Although all of these dosage forms are convenient to use, some of them, such as paste, lozenge, troche, solution and gel, may be considered even more advantageous due to the relative ease with which they can be applied at a specific site in the oral cavity with a finger tip, or the ease with which they can be easily masticated for contact with the oral mucosa.

An important physical characteristic for all dosage forms is the rate of release of the treating agent(s); from the dosage form. It is known, for example, that tablets containing nitroglycerin are formulated to disintegrate quickly under the tongue for immediacy in drug availability to ameliorate anginal pain. This characteristic may not, however, be desirable for all drugs. In drugs which are intended for a topical mode of action, or of a drug which may possess a relatively low rate of absorption through the oral mucosa, it would be desirable to release the drug slowly from the dosage form. Such a slow release rate would minimize swallowing of the drug and would also lower or eliminate the occurence of pharmacological or toxicological side effects. It is also known that generally a solubilized version of the drug is absorbed faster through the skin and mucous membrane than the solid version. In the case of the treating agents that are the subject of this application, it was decided to formulate both versions to provide both types of release rates.

Oral paste formulas and troches contain solid crystalline treating agents. Mouthwash formulas contain the solubilized version of treating agents.

## Oral Paste

The major criteria of a paste for use in the oral cavity are as follows: (1) the paste should adhere to the mucous membrane until the desired amount of treating agent(s) has been released; (2) the paste should not move from its site of application; (3) the paste should be composed of safe, edible excipient; (4) it should not alter the taste or should not leave an aftertaste in the mouth; (5) it should be viscous enough to facilitate the application via fingertip, and; (6) it should be homogeneous and non-gritty.

Paste formulas may contain the following categories of ingredients: (1) diluents (also known as fillers) such as dicalcium phosphate, lactose and starch; (2) adhesives that provide adhesion in the presence of saliva and moisture such as gelatin, pectin, acacia gum, xanthan gum and starch derivatives; (3) viscosity builders such as microcrystalline wax, carboxymethylcellulose sodium (abbreviated as CMC-Na or CMC Sod. or CMC, cross-linked carboxymethylcellulose sodium, petrolatum and polyethylene polymer; (4) plasticizers such as mineral oil and vegetable oils such as olive, safflower, peanut, sesame and sweet almond oil; (5) anionic or nonionic emulsifiers or wetting agents such as glyceryl monostearate, sodium stearate, polysorbate 60, polysorbate 80, Ceteth-20, Steareth-20 and Laureth-23; (6) flavoring agents such as fruit or vegetable flavors, vanilla and chocolate; (7) sweetening agents such as sucrose, saccharin sodium, cyclamate and aspartame; (8) antibacterial preservatives such as benzyl alcohol and sodium benzoate; (9) taste modifiers such as sodium ascorbate, citric acid and sodium tartrate, and; (10) coloring or opaquing agents such as edible colors, dyes and titanium dioxide.

## Mouthwash

One or more treating agents may be solubilized and formulated into a mouthwash to provide a limited amount of treating agent in a pleasant-tasting, flavored vehicle which may be used more than once during the day. A distinct advantage of a mouthwash resides in its ability to reach deeper crevices between teeth and the distant areas of the mouth which are inaccessible to the fingertips for a comfortable or convenient mode of

application. It is not difficult for a patient to gargle with the medicated mouthwash to provide a treating agent or combination of treating agents to the deeper areas of the throat.

The following ingredients may be included in a mouthwash formula: (1) diluents such as plain water or flavored water; (2) solvents such as glycerin, ethanol, propylene glycol and other polyols; (3) buffering agents such a sodium citrate and sodium phosphate; (4) organic acids such as citric, phosphoric or tartaric acid; (5) sweeteners such as sucrose, saccharine sodium, cyclamate or aspartame; (6) flavoring agents; (7) coloring agents; (8) preservatives such as sodium benzoate and benzyl alcohol; (9) inorganic acids such as hydrochloric or phosphoric to adjust the pH; (10) water soluble salts such as sodium chloride as taste modifier, and zinc chloride/citrate as astringent, and; (11) antibacterial agents such as cetylpyridinium chloride or benzalkonium chloride at appropriate concentration as allowed by the regulatory authorities.

## Troches

Also known as lozenges or pastilles, troches are round disc-shaped solids containing the treating agent(s) in a suitable flavored base. The base may be preferably glycerinated gelatin or a mixture of sugar and a mucilagenous gum such as acacia or tragacanth. The treating agent(s) may be dispersed at a concentration between 0.1% to 10.0% by weight in a mixture of powdered sugar and powdered acacia or tragacanth. The mucilagenous gum (acacia or tragacanth) may be incorporated in the formula at a concentration of 2% to 10% by weight. The preferred concentration of acacia or tragacanth is between 5% and 8% by weight. This concentration of gum gives sufficient adhesiveness to the mass. The mass is formed by slowly adding water to the mixture of powdered sugar, amlexanox and powdered gum. The water is added until a pliable mass is formed. The mass is rolled out on a clean glass plate and the troche pieces are cut out using a cutter. The mass may be otherwise rolled into a cylinder and divided into pieces of desirable weight. Each piece is then shaped and allowed to dry before packing. Alternate suitable mechanical means may be employed to produce such troches containing one or more treating agents.

The following examples serve to illustrate the method of the invention without restricting said process.

## EXAMPLE 1

Table 1 sets forth several paste formulations that were found useful in practicing the method described herein.

TABLE 1

| FORMULA FOR VEHICLE PASTE | (A) | (B) | (C) | (D) | (E) | (F) | (G) | (H) | (I) |
|---|---|---|---|---|---|---|---|---|---|
| Mineral Oil | 27.3 | 26.3 | 38.3 | 36.3 | 46.0 | 28.8 | 28.8 | 47.5 | 32.2 |
| Gelatin | 17.5 | 17.5 | 18.5 | 18.4 | 12.5 | 18.4 | 18.4 | 20.0 | 17.5 |
| Pectin | 17.5 | 17.5 | 18.5 | 18.3 | 12.5 | 18.4 | 18.4 | 20.0 | 17.5 |
| Petrolatum | 11.4 | 11.4 | | 5.0 | | | 3.3 | | |
| Cross-linked Carboxy-methylcellulose sodium | | | | 18.3 | 12.5 | | | 10.0 | |
| Carboxymethylcellulose sodium (7HF) | 8.7 | 8.7 | 9.2 | | | 9.2 | 9.2 | | 8.7 |
| Carboxymethylcellulose sodium (7MF) | 8.7 | 8.7 | 9.2 | | | 9.2 | 9.2 | | 8.7 |
| Microcrystalline wax | | | | | | 6.7 | 3.4 | | 12.8 |
| Glyceryl monostearate | 6.4 | 6.4 | | | | 6.7 | 6.7 | | |
| Polyethylene | | | 4.3 | 3.6 | 4.0 | | | 2.5 | |
| Xanthan gum | | | | | 12.5 | | | | |
| Titanium dioxide | | 1.0 | 2.0 | | | | | | 1.1 |
| Benzyl alcohol | 2.5 | 2.5 | | | | 2.6 | 2.6 | | 1.5 |
| Flavor, Sweetner | qs | qs | qs | qs | qs | | | | |

**EXAMPLE 2**

The following ranges of excipients (percent weight/weight) were found useful in vehicle pastes.

| Excipient | Percent w/w Range |
|---|---|
| Mineral Oil | 27.0 - 47.5 |
| Gelatin | 12.0 - 20.0 |
| Pectin | 12.0 - 20.0 |
| Petrolatum | 3.0 - 11.5 |
| Cross-linked CMC Sodium | 10.0 - 20.0 |
| CMC Sodium (7HF) | 8.0 - 10.0 |
| CMC Sodium (7MF) | 8.0 - 10.0 |
| Microcrystalline wax | 3.0 - 7.0 |
| Glyceryl monostearate | 3.0 - 10.0 |
| Polyethylene | 2.0 - 4.5 |
| Xanthan gum | 1.0 - 15.0 |
| Titanium dioxide | 0.1 - 3.0 |
| Benzyl alcohol | 0.5 - 3.0 |

Final Composition of Paste

| Ingredient | PERCENT WEIGHT/WEIGHT | | | | |
|---|---|---|---|---|---|
| TREATING AGENT(S) | 10.0 | 7.5 | 5.0 | 2.5 | 0.1 |
| VEHICLE | 90.0 | 92.5 | 95.0 | 97.5 | 99.9 |

**Method of Preparation of Oral Paste**

Screen all the solids through a suitable sieve such as 60 or 70 mesh sieve and then mix the preweighed amounts in a suitable blender such as a V-Blender until adequately mixed. In a separate suitable vessel add weighed formula amounts of mineral oil, petrolatum, surfactant such as glyceryl monostearate, polysorbates and polyethylene. Heat this vessel with constant stirring until a homogeneous fluid is obtained. While slowly cooling with continuous stirring add the blended solids and keep stirring to obtain a homogeneous dispersion of solids in the oil phase. At 45°-50° C add the preservatives,and cool down to room temperature with continuous stirring. Pass the final semisolid product through an ointment roller mill to homogenize the product.

**EXAMPLE 3**

The formulas described below represent particularly preferred mouthwash formulations:

| Ingredient | Percent weight/weight | | |
|---|---|---|---|
| Water | 81.7 | 82.3 | 83.2 |
| Ethanol | 12.8 | 12.8 | 12.8 |
| Glycerin | 3.0 | 3.0 | 3.0 |
| Sodium citrate | 0.2 | - | - |
| Citric acid | 0.2 | - | - |
| Triethanolamine | 1.0 | 0.7 | 0.7 |
| Treating Agent(s) | 1.0 | 1.0 | 1.0 |
| Flavoring agent | 0.1 | 0.1 | 0.1 |
| Coloring agent | q.s. | q.s. | q.s. |
| Hydrochloric acid | - | q.s. to pH 7.5 | q.s. to pH 7.5 |

The above mentioned ingredients may be used in the percent (w/w) range described below to obtain a more suitable version:

| Ingredient | Percent w/w range |
|---|---|
| Water | 60.0 - 95.0 |
| Ethanol | 8.0 - 15.0 |
| Glycerin | 1.5 - 6.5 |
| Sodium citrate | 0.1 - 0.9 |
| Citric acid | 0.1 - 1.0 |
| Triethanolamine | 0.1 - 1.5 |
| Treating Agent(s) | 0.1 - 1.5 |
| Flavoring agent | 0.1 - 1.0 |
| Coloring agent | 0.1 - 1.0 |
| Astringent Salt | 0.1 - 1.5 |

## Method of Preparation Of Mouthwashes

To a suitable vessel add the formula amount of treating agent(s) and add designated amount of triethanolamine. Stir to mix well. To this mixture, add while stirring, ethanol, glycerin, water, buffering agents, flavors and colors. Stir well to mix.

## EXAMPLE 4

The following paste formulations were tested and found useful in the treatment of aphthous ulcers:

Formula No. 04-27a(A)

| | |
|---|---|
| AC-9 Homopolymer(polyethylene; Allied) | 3.8% |
| Kaydol Mineral Oil (viscosity 340-355) | 38.9% |
| Pectin, USP | 17.4% |
| Gelatin, NF | 18.1% |
| CMC 7MF(Na; Hercules; Aqualon) | 8.7% |
| CMC 7HF(Na; Hercules; Aqualon) | 8.7% |
| Treating Agent(s) | 5.0% |

Formula No.: 04-27a(C)

| | |
|---|---|
| AC-9 Homopolymer(polyethylene; Allied) | 4.2% |
| Kaydol Mineral Oil (viscosity 340-355) | 38.3% |
| Pectin, USP | 17.4% |
| Gelatin, NF | 17.4% |
| CMC Na 7MF(Hercules) | 8.7% |
| CMC 7HF(Hercules) | 8.7% |
| Treating Agent(s) | 5.0% |

**Paste Manufacturing Procedure**

1) Screen gelatin to a fine mesh (preferably 100 mesh sieve).
2) Mechanically grind pectin to a fine powder.
3) To a blender add pectin, gelatin, CMC 7HF and CMC 7MF
4) To a separate container add AC-9 homopolymer and mineral oil and heat to 90 C or until mixture is clear and homogeneous.
5) While hot, add step 3 to step 4 with stirring and cool to room temperature with constant stirring.
6) Mill the mixture from step 5 on a 3-roll mill until homogeneous

This formulation appears to be physically stable after aging 2 weeks at 40 C.

**EXAMPLE 5**

**STUDY SUMMARY**

**OBJECTIVE**

The objective of this clinical study was to evaluate the tolerance and efficacy of 5% of a treating agent according to the invention adhesive paste when applied to patients with aphthous ulcers.

**STUDY PLAN**

This 4-day clinical study utilized a double-blind, randomized, uneven parallel-group, multi-center design.

Thirty-five patients with aphthous ulcers who met all of the inclusion and none of the exclusion criteria were enrolled into this study. Patients were treated on the buccal mucosa, oral labial mucosa, floor of mouth or the distal half of the tongue.

Patients enrolled into the study were treated with either the 5% of a treating agent according to the invention adhesive paste or the vehicle adhesive paste. The study drug was applied to a maximum of 3 ulcers identified

for treatment twice daily for 3 days. Tolerance and efficacy evaluations were made twice daily and again in the morning of the fourth day.

## RESULTS

### Demographic and Background Data

Data consisted of information from 35 patients who were treated with either 5% of a treating agent (t.a.) adhesive paste or the vehicle.

| Demographics | | | |
|---|---|---|---|
| Study Drug | Total Patients Enrolled | No. Safety Analysis | No. Efficacy Analysis |
| 5% t.a. | 21 | 21 | 18 |
| Vehicle | 14 | 14 | 14 |
| Total | 35 | 35 | 32 |

### Efficacy

The efficacy data was generated from an evaluation of the signs and symptoms of the patients with the aphthous ulcers. The evaluations included a measurement of ulcer size, the severity of erythma and pain, and an evaluation of overall improvement. The evaluations were made twice daily for three days and again on the morning of the fourth day. The following table summarizes the results from all study sites.

| Summary of Efficacy Evaluations | | | | | |
|---|---|---|---|---|---|
| | Median | | Mean | | |
| Evaluation | 5% t.a. | Vehicle | 5% t.a. | Vehicle | p.value |
| % Reduction in Pain | 93% | 57% | 73% | 61% | |
| % Reduction in Size | 88% | 37.5% | 69% | 41% | |
| Erythema* | -4 | 1-.5 | -2.9 | -1.4 | |
| Improvement† | 3 | 0.5 | 2.4 | 0.9 | <0.0001 |

*ERYTHEMA ADJUSTMENT SCALE

| | |
|---|---|
| -4 = No Erythema | 0 = No Change from Day 1 AM |
| -3 = Marked Decrease | 1 = Slight Increase |
| -2 = Moderate Decrease | 2 = Moderate Increase |
| -1 = Slight Decrease | 3 = Marked Increase |

PHYSICIAN'S IMPROVEMENT SCALE

| Grade | | Description of Ulcer |
|---|---|---|
| 4 | = | Aphthous ulcer cleared |
| 3 | = | Marked improvement (the ulcer is barely perceptible with minimal or no pain and marked decrease in size) |
| 2 | = | Moderate improvement (the ulcer is visible with moderate decrease in erythema and a moderate decrease in pain and moderate decrease in size) |
| 1 | = | Slight improvement (the ulcer is visible with a slight decrease in size, minimal decrease in erythema and a slight decrease in pain) |
| 0 | = | No change from the A.M. Day 1 |
| -1 | = | Aphthous ulcer worsened (greater erythema and/or pain or size) |

## Safety

The safety of the 5% treating agent oral paste was assessed on the basis of the incidence, nature and severity of the adverse experiences reported during the study. During the conduct of this study no adverse experiences were reported.

## Discontinuations

A total of 3 patients discontinued therapy with the 5% treating agent oral paste. All three patients discontinued due to conflicts in scheduling.

## Conclusion

Patients with aphthous ulcers who had been treated twice-a-day for three days with 5% treating agent showed clinically significant improvement in all parameters measured over the vehicle paste. Statistical significance was seen in the measurements of ulcer size, reduction in erythema and overall improvement.

No adverse reactions of any type were reported by either the patient or the investigator during the study.

## Claims

1. A method of treating aphthous ulcers and other mucocutaneous disorders comprising contacting said aphthous ulcer or other mucocutaneous disorder with an amount of a composition containing an effective amount of a treating agent comprising one or more compounds having one or more of the following biochemical/biological properties: mediator release inhibitors; 5-lipoxygenase inhibitors; leukotriene antagonists; and, platelet-activating factor antagonists.

2. The method according to claim 1 wherein the concentration of the treating agent is from about 0.1% to about 10% of the composition.

3. The method according to claim 1 wherein the treating agent comprises one or more compounds having the biochemical/biological property of a mediator release inhibitor.

4. The method according to claim 3 wherein the compounds having the biochemical/biological property of a mediator release inhibitor are selected from the group consisting of the following:

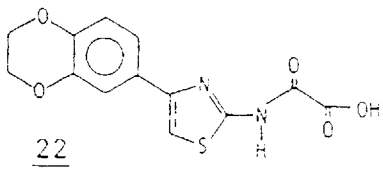
22

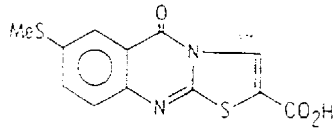
23

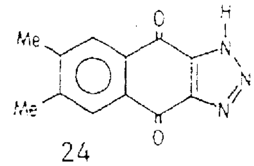
24

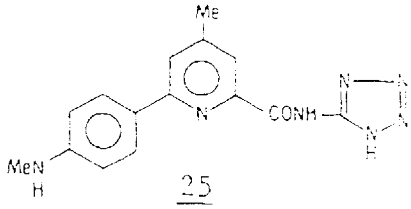
25

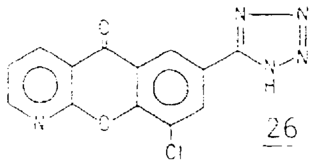
26

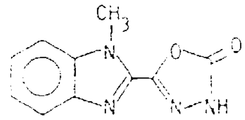
27

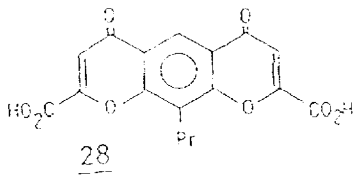
28

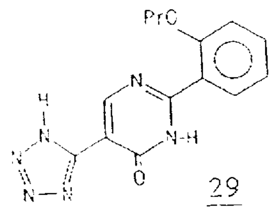
29

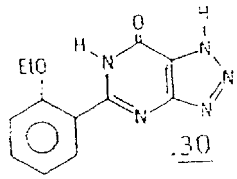
30

5. The method according to claim 1 wherein the treating agent comprises one or more compounds having the biochemical/biological property of a 5-lipoxygenase inhibitor.

6. The method according to claim 5 wherein the compounds having the biochemical/biological property of a 5-lipoxygenase inhibitor are selected from the group consisting of the following:

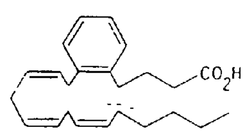
1

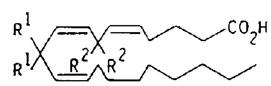
2 a) $R^2 = CH_3$, $R^1 = H$
b) $R^1 = CH_3$, $R^2 = H$

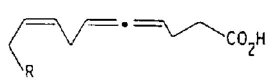
3 R = 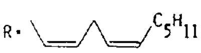
4 R = $C_{10}H_{21}$

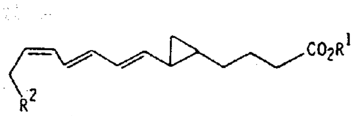
5 $R^2$ = 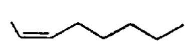 , $R^1 = H$
6 $R^2$ = 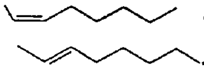 , $R^1 = CH_3$

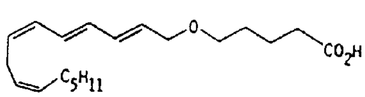
7

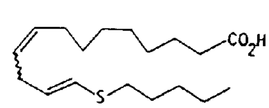
8

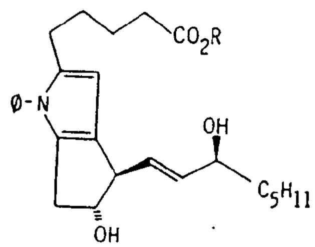

9

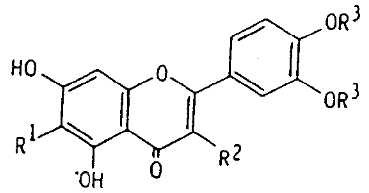

10 $R^1 = R^3 = H$, $R^2 = OH$

11 $R^1 = OCH_3$, $R^2 = H$, $R^3 = CH_3$

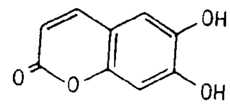

12

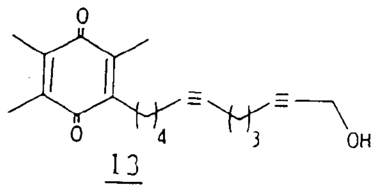

13

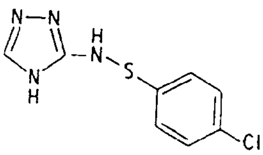

14

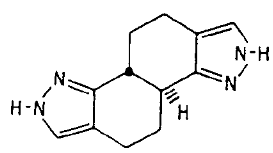

15

7. The method according to claim 1 wherein the treating agent comprises one or more compounds having the biochemical/biological property of a leukotriene antagonist.

8. The method according to claim 7 wherein the compounds having the biochemical/biological property of a leukotriene antagonist are selected from the group consisting of the following:

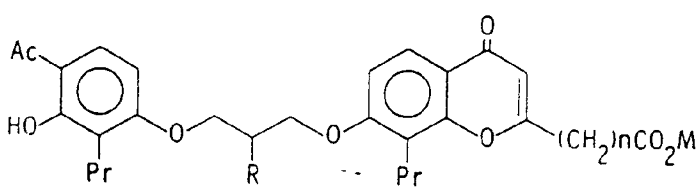

16 R=OH n=0 M=Na

17 R=H n=2 M=(Lysine)H

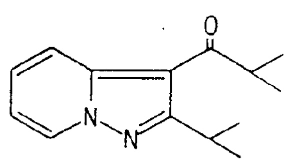

18

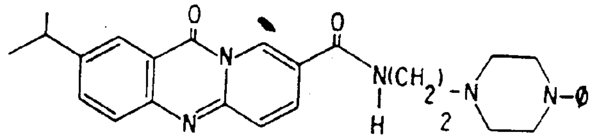

19

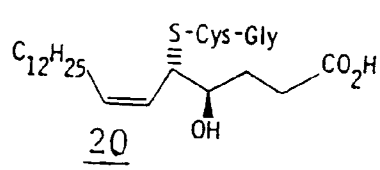

20

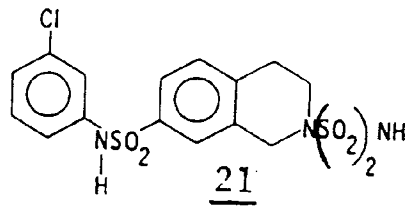

21

9. The method according to claim 1 wherein the treating agent comprises one or more compounds having the biochemical/biological property of a platelet-activating factor antagonist.

10. The method according to claim 9 wherein the compounds having the biochemical/biological property of a platelet-activating factor antagonist are selected from the group consisting of the following:

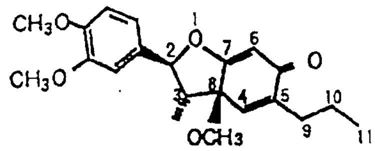

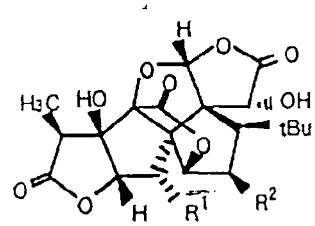

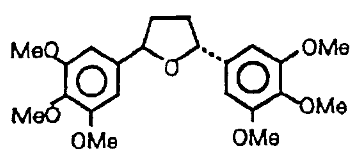

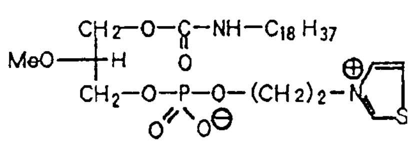

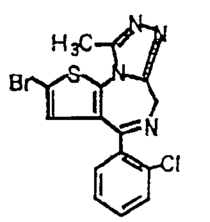

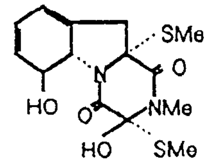

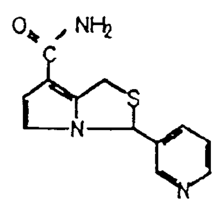

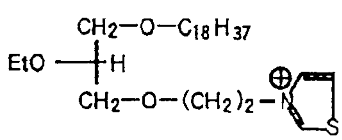

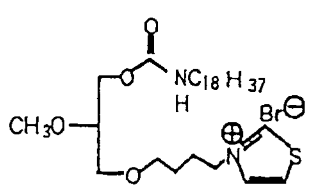

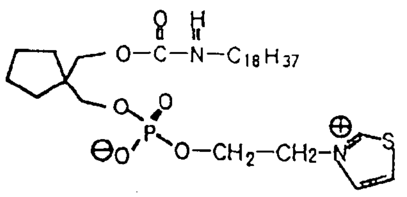

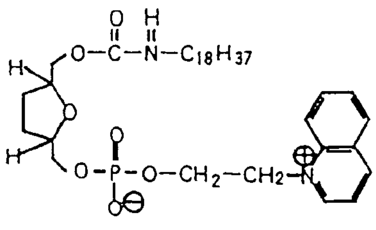

**11.** The method according to claim 1 wherein the treating agent is of the formula:

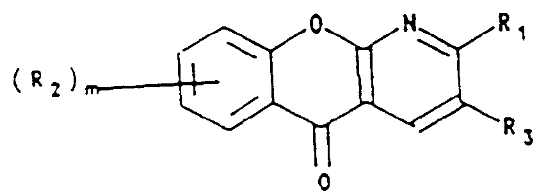

wherein $R_1$ is hydrogen, alkyl, phenyl, carboxyl, hydroxyl, alkoxy, carboxyalkyl (i.e. esters), cyano, acylamino, or amino group which may be unsubstituted or substituted by up to two alkyl groups; m is 0, 1 or 2 and $R_2$ is alkyl, alkenyl, alkoxy, halogen, nitro, hydroxy, carboxyl, butadienylene (-CH=CH-CH=CH-) which forms a benzene ring with any adjacent carbon atoms, cyano, carboxyalkyl, trifluoromethyl, or amino group which may be unsubstituted or substituted by at least one alkyl; and $R_3$ is carboxyl, cyano, arylalkoxycarbonyl, alkoxycarbonyl, or carboxamide which may be unsubstituted or substituted by at least one alkyl, and the salts thereof.

**12.** The method according to claim 11 wherein $R_1$ is an amino group optionally substituted with alkyl or acyl groups, m is 1, $R_2$, is lower alkyl, and $R_3$ is carboxyl, and the salts thereof.

**13.** The method according to claim 12 wherein $R_1$ is amino, m is 1, $R_2$ is 7-isopropyl, $R_3$ is carboxyl, and the salts thereof.

**14.** The method according to claim 1 wherein said contacting is by means of topical application.

**15.** The method according to claim 1 wherein the composition comprises the treating agent and a vehicle selected from the group consisting of a paste, solution, gel, quick-disintegrating tablet, mouthwash, ointment, cream, powder, adhesive patch, aerosolized spray, lozenge, troche, dentifrice, and dental floss.

**16.** The method according to claim 15 wherein the composition comprises about 90 to about 99.9% of a paste vehicle and about 0.1% to about 10% of the treating agent.

**17.** The method according to claim 16 wherein the paste vehicle comprises about 27% to about 48% mineral oil, about 12% to about 20% gelatin, about 12% to about 20% pectin, about 3% to about 12% petrolatum, about 10% to about 20% cross-linked sodium carboxymethylcellulose, about 8% to about 10% 7HF, about 8% to about 10% 7MF, about 3% to about 7% microcrystalline wax, about 3% to about 10% glyceryl monostearate, about 2% to about 5% polyethylene, about 1% to about 15% xanthan gum, about 0.1% to about 3% titanium dioxide and about 0.5% to about 3% benzyl alcohol.

**18.** The method according to claim 15 wherein the composition comprises about 98.5% to about 99.9% of a mouthwash vehicle and about 0.1 to about 1.5% of said treating agent.

**19.** The method according to claim 18 wherein the mouthwash vehicle comprises about 60% to about 95% water, about 8% to about 15% ethanol, about 1.5% to about 6.5% glycerin, about 0.1% to about 0.9% sodium citrate, about 0.1% to about 1.5% triethanolomine, about 0.1% to about 1.0% of a flavoring agent, about 0.1% to about 1.0% of a coloring agent and about 0.1% to about 1.5% of an astringent salt, wherein all percentages are on a w/w basis.

**20.** The method according to claim 15 wherein the composition is a troche.